# EUROPÄISCHE PATENTSCHRIFT

(11) **EP 1 107 947 B1**
(45) Veröffentlichungstag und Bekanntmachung des Hinweises auf die Patenterteilung: **08.01.2003**
(21) Anmeldenummer: 99946100.7
(22) Anmeldetag: 02.09.1999
(51) Int. Cl.: C07C 255/19

(54) **VERFAHREN ZUR HERSTELLUNG VON CYANVALERIANSÄURE ODER -ESTERN**
METHOD FOR PRODUCING CYANOVALERIC ACID OR ESTERS THEREOF
PROCEDE DE PREPARATION D'ACIDE OU D'ESTERS CYANOVALERIANIQUE(S)

(30) Priorität: 03.09.1998 DE 19840253
(43) Veröffentlichungstag der Anmeldung: 20.06.2001
(73) Patentinhaber: BASF AKTIENGESELLSCHAFT, 67056 Ludwigshafen (DE)
(72) Erfinder: SLANY, Michael, D-67281 Kirchheim (DE); SCHÄFER, Martin, D-67063 Ludwigshafen (DE); SCHULZ, Michael, D-67067 Ludwigshafen (DE)
(74) Vertreter: Kinzebach, Werner, Dr.
(86) Internationale Anmeldenummer: EP9906463
(87) Internationale Veröffentlichungsnummer: WO00014055

(56) Entgegenhaltungen:
- EP-A- 0 227 160
- EP-A- 0 377 838
- EP-A- 0 495 547
- WO-A-97/08127
- US-A- 4 257 973

## Beschreibung

Die vorliegende Erfindung betrifft ein Verfahren zur Herstellung von Cyanvaleriansäure oder deren Estern durch Umsetzung von 2-, 3- oder 4-Pentennitril oder Gemischen hiervon mit Kohlenmonoxid und einer hydroxylgruppenhaltigen Verbindung in Gegenwart eines Katalysatorsystems.

5-Cyanvaleriansäure und ihre Ester sind wertvolle Ausgangsstoffe für die Herstellung von Farbstoffen, Schädlingsbekämpfungsmitteln, Fasern, insbesondere Polyamidfasern und Kunststoffen. Durch Hydrierung zu 6-Aminocapronsäure bzw. 6-Aminocapronsäureestern und Abspaltung des Wassers oder Alkohols erhält man Caprolactam.

Die Herstellung von Cyanvaleriansäure und Cyanvaleriansäureestern durch Carbonylierung von Pentennitrilen in Gegenwart von Katalysatoren ist bekannt. Die bekannten Synthesen erfolgen durch Umsetzung von einem Pentennitril mit Kohlenmonoxid in Anwesenheit von Wasser oder Alkohol unter erhöhter Temperatur und erhöhtem Druck in Gegenwart eines Katalysatorsystems. Als Katalysatorsysteme finden überwiegend Cobaltverbindungen, wie Co₂(CO)₈ oder Co(OAc)₂, unter Mitverwendung von Stickstoffbasen (siehe GB-1 497 046 und DE-2 541 640), speziellen Lösungsmitteln, wie Sulfolan (siehe US 4 508 660), cyclischen Amid- oder Harnstoffderivaten (siehe EP-373 579) oder Nitrilen (siehe EP-377 838 und US 4 933 483) Verwendung.

Die EP 0 450 577 beschreibt ein Rh/HI-Katalysatorsystem zur Hydroxycarbonylierung von Pentennitril zu Cyanvaleriansäure.

Nitrilgruppen enthaltende Verbindungen bilden häufig Komplexe mit Übergangsmetallen. Katalytisch aktive Katalysatorkomplexe neigen dazu, durch die Nitril-Koordination desaktiviert zu werden. Dies gilt besonders für Palladium, da Palladium sehr leicht stabile Nitril-Komplexe bildet, wie z. B. (PhCN)₂PdCl₂, (CH₃CN)₂PdCl₂. Die palladiumkatalysierte Carbonylierung von nitrilgruppenhaltigen Olefinen verläuft daher im Allgemeinen nur mit sehr niedriger Katalysatoraktivität. So beschreibt die US 4 257 973 die Carbonylierung von 3-Pentennitril mit dem Katalysatorsystem (Ph₃P)₂PdCl₂/SnCl₂. Die Reaktion verläuft mit unbekannter Selektivität und die Ausbeute an nicht genau angegebenen Cyansäureestern betrug lediglich 5 % (siehe Beispiel 108 der US 4 257 973).

Die EP 0 495 547 beschreibt ein Verfahren zur Monocarbonylierung gegebenenfalls substituierter olefinisch ungesättigter Verbindungen in Gegenwart eines Katalysatorsystems, das Pd-Kationen, einen zweizähnigen Diphosphin-Liganden und eine Anionenquelle umfasst. Nach der Beschreibung der EP 0 495 547 kann das Ausgangsolefin z. B. mit Cyano- oder Nitrilgruppen substituiert sein. Soweit Alkensäurederivate, wie Alkensäurenitrile, als Ausgangsstoffe verwendet werden, soll das Alkensäurederivat vorzugsweise ein 2-Alkensäurederivat sein. Bei der Carbonylierung von Alkensäurederivaten umfasst das Katalysatorsystem vorzugsweise einen Promotor, z. B. Chinone und Nitroverbindungen. In Beispiel 59 der EP 0 495 547 wird die Carbonylierung von Acrylnitril mit Kohlenmonoxid und Methanol in Gegenwart von Pd(OAc)₂, TBPD (1,3-Bis(din-butylphosphino)propan), NiTFS (Nickel-di-trifluormethylsulfonat) und 1,4-Naphthochinon beschrieben. Die Umwandlung zum Monomethylester-Mononitril der Malonsäure erfolgte jedoch nur zu 5 %, bezogen auf das eingesetzte Acrylnitril. In Kenntnis der EP 0 495 547 hätte der Fachmann für die Umsetzung anderer nitrilsubstituierter Olefine als Acrylnitril nach dem dort beschriebenen Verfahren noch niedrigere Ausbeuten erwartet.

Es wurde nun überraschenderweise gefunden, dass die Carbonylierung von 2-, 3- oder 4-Pentennitril mit einem Katalysatorsystem, das dem der EP 0 495 547 vergleichbar ist, in hoher Ausbeute und hoher Selektivität gelingt. Außerdem wurden neue Katalysatorsysteme und neue Diphosphin-Liganden gefunden, mit denen die Ausbeute und Selektivität weiter gesteigert werden können.

Demzufolge betrifft die vorliegende Erfindung ein Verfahren zur Herstellung von Cyanvaleriansäure oder deren Estern durch Umsetzung von 2-, 3- oder 4-Pentennitril oder Gemischen hiervon mit Kohlenmonoxid und einer Hydroxylgruppen-haltigen Verbindung in Gegenwart eines Katalysatorsystems, das umfasst: (i) eine Palladium(II)-Verbindung (ii) einen zweizähnigen Diphosphinliganden und (iii) eine Anionenquelle.

Als Ausgangsstoffe für die erfindungsgemäße Carbonylierung werden 2-, 3- oder 4-Pentennitril oder Gemische hiervon eingesetzt. Die Verwendung von 3- und/oder 4-Pentennitril bzw. von Gemischen, die 3- und/oder 4-Pentennitril als Hauptkomponenten enthalten, ist bevorzugt. 3-Pentennitril oder Gemische mit 3-Pentennitril als Hauptkomponente sind am meisten bevorzugt. 3-Pentennitril lässt sich z. B. durch Addition von Blausäure an Butadien, beispielsweise in Gegenwart von nickelhaltigen Komplexverbindungen oder Kupfer(I)-Chlorid nach den in den deutschen Offenlegungsschriften 1 593 277, 2 344 767 und 2 009 470 beschriebenen Verfahrensweisen, herstellen.

Beim erfindungsgemäßen Verfahren wird mit hoher Selektivität 5-Cyanvaleriansäure erhalten, ungeachtet, ob 2-, 3- oder 4-Pentennitril oder ein Gemisch davon eingesetzt wird. Beim Einsatz von 2- oder 3-Pentennitril erfolgt vermutlich eine vorgelagerte Isomerisierung zu 4-Pentennitril. Die Carbonylierung von 4-Pentennitril zu 5-Cyanvaleriansäure(ester) nach dem erfindungsgemäßen Verfahren erfolgt weitgehend regioselektiv mit Selektivitäten, die im Allgemeinen über 70 %, vorzugsweise über 80 %, bezogen auf gebildete Cyanvaleriansäure(ester), liegen.

Bei der Palladium(II)-Verbindung handelt es sich vorzugsweise um ein Palladiumsalz. Beispiele geeigneter Palladiumsalze sind unter anderem die Salze der Salpetersäure, Schwefelsäure, von Sulfonsäuren, z. B. Chlorsulfonsäure, Methansulfonsäure, Trifluormethansulfonsäure, t-Butylsulfonsäure, p-Toluolsulfonsäure, oder eines sulfonierten Ionenaustauscherharzes, oder einer Carbonsäure, z. B. einer Alkansäure, wie Essigsäure oder Trifluoressigsäure. Es versteht sich, dass wenn es sich bei der Palladium(II)-Verbindung um ein Palladiumsalz einer geeigneten Säure handelt, diese Verbindung gleichzeitig die erfindungsgemäß zu verwendende Anionenquelle darstellen kann. Die Palladium(II)-Verbindung kann ferner in Form eines Palladiumkomplexes, z. B. eines Komplexes mit einem zweizähnigen Diphosphinliganden, vorliegen. In diesem Fall enthält die Palladium(II)-Verbindung gleichzeitig den erfindungsgemäß zu verwendenden zweizähnigen Diphosphinliganden. Die Palladium(II)-Verbindung kann auch ausgehend vom elementaren Zustand in situ gebildet werden.

Die Menge an Palladium(II)-Verbindung ist nicht kritisch. Vorzugsweise beträgt die Menge 10⁻⁷ bis 10⁻¹ Mol Pd pro Mol eingesetztes Pentennitril, insbesondere 10⁻⁶ bis 10⁻².

Der zweizähnige Diphosphinligand kann als solcher oder in Form eines Komplexes mit der Palladium(II)-Verbindung eingesetzt werden. Vorzugsweise weist der Diphosphinligand folgende allgemeine Strukturformel auf:

R¹R²P-X-PR³R⁴

worin R¹, R², R³ und R⁴ unabhängig voneinander für C₁- bis C₂₀-Alkyl, C₃- bis C₁₀-Cycloalkyl, Aryl oder Heteroaryl mit bis zu 4 kondensierten aromatischen Ringen oder C₇- bis C₂₀-Aralkyl, die gegebenenfalls substituiert sein können, oder R¹ und R² gemeinsam und/oder R³ und R⁴ gemeinsam für C₂- bis C₂₀-Alkylen, Arylen oder Heteroarylen mit bis zu 4 aromatischen Ringen, C₇- bis C₂₀-Aralkylen stehen, die gegebenenfalls substituiert sein können, und X für einen zweiwertigen verbrückenden Rest steht, wobei die flankierenden Phosphoratome durch 1 bis 10 Atome getrennt sind.

Stärker bevorzugte Diphosphinliganden sind dadurch gekennzeichnet, dass R¹, R², R³ und R⁴ unabhängig voneinander jeweils für einen unsubstituierten, gegebenenfalls verzweigten, gegebenenfalls cyclischen Alkylrest mit 1 bis 10 Kohlenstoffatomen stehen oder R¹ und R² gemeinsam und/oder R³ und R⁴ gemeinsam einen gegebenenfalls verzweigten, gegebenenfalls cyclischen Alkylenrest mit 1 bis 10 Kohlenstoffatomen ausbilden.

In besonders bevorzugten Ausführungsformen sind R¹, R², R³ und R⁴ ausgewählt unter Methyl-, Ethyl-, Propyl-, Isopropyl-, n-Butyl-, s-Butyl, t-Butyl- und/oder Cyclohexylresten oder bilden R¹ und R² gemeinsam und/oder R³ und R⁴ gemeinsam einen Pentamethylen, Hexamethylen- oder Cyclooctylenrest aus. Soweit vorstehend angegeben ist, dass die Reste R¹, R², R³ und R⁴ gegebenenfalls substituiert sein können, kann es sich bei den Substituenten um beliebige Substituenten handeln, die die katalytische Aktivität des Systems nicht beeinträchtigen. Geeignete Substituenten sind unter anderem Halogenatome, Alkoxygruppen, Halogenalkylgruppen, Halogenalkoxygruppen, Acylreste, Acyloxygruppen, Aminogruppen, Hydroxylgruppen, Nitrilgruppen, Acylaminogruppen und Arylgruppen.

Der erfindungsgemäß zu verwendende Diphosphinligand ist zweizähnig, d. h. er muss die zwei Phosphin-Phosphoratome in einer intramolekularen Entfernung und Konfiguration enthalten, die die Ausbildung einer koordinativen Bindung beider Phosphoratome zu einem einzelnen Palladiumatom gestatten. In der obigen bevorzugten Strukturformel steht X daher für einen zweiwertigen verbrükkenden Rest, wobei die flankierenden Phosphoratome durch 1 bis 10 Atome getrennt sind. Die verbrückende Gruppe enthält vorzugsweise keine Substituenten, die die Koordinierung sterisch hindern. Vorzugsweise steht X für eine gegebenenfalls Heteroatome enthaltende Alkylenkette, wie einen zweiwertigen Kohlenwasserstoff, Etheroder Thioetherrest. Beispiele verbrückender Gruppen X sind z. B. -CH₂-, -CH₂CH₂-, -CH₂CH₂CH₂-, -CH₂OCH₂-, -CH₂SCH₂-, -CH₂CH₂CH₂CH₂-, -CH₂CH₂OCH₂CH₂-, -CH₂CH₂SCH₂CH₂-, -CH₂CH₂OCH₂CH₂OCH₂CH₂-, -CH₂C(CH₃)₂-CH₂-, -CH₂-CH(CH₃)-O-CH(CH₃)-CH₂-.

Beispiele geeigneter zweizähniger Diphosphinliganden sind demzufolge: 1,2-Bis(di-n-butylphosphino)ethan, 1,3-Bis(dimethylphosphino)propan, 1,3-Bis(di-i-propylphosphino)propan, 1,3-Bis(din-propylphosphino)propan, 1,3-Bis(di-i-butylphosphino)propan, 1,3-Bis(di-n-butylphosphino)propan, 1,3-Bis(di-s-butylphosphino)propan, 1,3-Bis(di-t-butylphosphino)propan, 1,3-Bis(din-hexylphosphino)propan, 1,2-Bis(di-cyclohexylphosphino)ethan, 1,3-Bis(n-butylmethylphosphino)propan, 1,3-Bis(n-butylethylphosphino)propan, 1,3-Bis(1,5-cyclooctylenphosphino)propan und sein 1,4-Cyclooctylengruppen enthaltendes isomeres Gemisch, 1,4-Bis(di-i-propylphosphino)butan, 1,5-Bis(dimethylphosphino)-3-oxapentan, 1,8-Bis(di-n-butylphosphino)-3,6-dioxaoctan und 1,4-Bis(di-n-butylphosphino)-2,2,3,3-tetramethylbutan.

In besonders bevorzugten Ausführungsformen steht X für

-CH₂-N(R⁵)-CH₂-,

worin R⁵ für Wasserstoff, geradkettiges oder verzweigtes C₁- bis C₂₀-Alkyl, wie Methyl, Ethyl, n-Propyl, i-Propyl, n-Butyl, s-Butyl, t-Butyl, Pentyl, Hexyl, Octyl, gegebenenfalls mit C₁- bis C₆-Alkyl substituiertes C₃- bis C₁₀-Cycloalkyl, einschließlich Bicycloalkyl, wie Cyclopropyl, Cyclopentyl, Cyclohexyl, Norbornyl, Pinanyl, Bornyl, Bicyclononyl, gegebenenfalls mit C₁- bis C₆-Alkyl substituiertes C₆- bis C₂₀-Aryl, wie Phenyl, Tolyl, Naphthyl, gegebenenfalls mit C₁- bis C₆-Alkyl substituiertes C₇- bis C₂₀-Aralkyl, z. B. mit 1 bis 6 C-Atomen im Alkylrest und 6 bis 14 C-Atomen im Arylrest, wie Benzyl, gegebenenfalls mit C₁- bis C₆-Alkyl substituiertes C₃- bis C₂₀-Heteroaryl, wie Pyridyl, Pyrimidyl, Pyrazinyl, Triazinyl, und C₂- bis C₂₁-Acyl steht.

Soweit die genannten Reste mit C₁- bis C₆-Alkyl substituiert sein können, können sie mit einer oder mehreren Alkylgruppen, z. B. Methyl oder Ethyl, substituiert sein.

Die vorgenannten Reste können mit einem oder mehreren, z. B. 1 bis 5, Substituenten, ausgewählt unter -NO, -NO₂, -CN, -CO₂⁻, -CO₂R⁶, -CONR⁶₂, Halogen, d.h. F, Cl, Br, I, -NR⁶₂, -OR⁶, -NR⁶⁺₃, -SO₃⁻, -SO₃R⁶, -SO₂R⁶, SO₂NR⁶₂ und SiR⁷₃ substituiert sein, wobei R⁶ für Wasserstoff, C₁- bis C₁₀-Alkyl, insbesondere Methyl, Ethyl oder i-Propyl, oder C₆- bis C₁₄-Aryl, insbesondere Phenyl, und R⁷ für C₁- bis C₁₀-Alkyl, insbesondere Methyl, Ethyl oder i-Propyl, oder C₆- bis C₁₄-Aryl, insbesondere Phenyl, stehen kann. Außerdem können in den vorgenannten Resten 1, 2, 3 oder 4 Kohlenstoffatome durch N oder O ausgetauscht sein.

R⁵ kann z. B. für die Gruppe -(CH₂)ₙ-Q stehen, die gegebenenfalls substituiert sein kann und Heteroatome, wie Sauerstoff oder Stickstoff, oder Aryleneinheiten, wie Phenylen, in der Alkylenkette enthalten kann, wobei Q für -SO₃⁻, CO₂⁻, CO₂R⁶, -CONR⁶₂, Halogen, -NR⁶₂, -OR⁶, NR⁶⁺₃ und n = 1-20, besonders bevorzugt 1-10, steht. R⁵ kann weiter für SiR⁷₃ stehen, wie Trimethylsilyl, t-Butyldimethylsilyl, Triphenylsilyl.

Es ist bevorzugt, dass R⁵ für einen elektronenziehenden Rest steht, da hierdurch die Stabilität der Diphosphinverbindung erhöht wird. Als elektronenziehende Reste R⁵ sind lineares oder verzweigtes C₁- bis C₂₀-Alkyl, das mit mindestens einer elektronenziehenden Gruppe substituiert ist, wobei sich die elektronenziehende Gruppe vorzugsweise in α-, β-, γ- und/oder δ-Position, insbesondere in α- und/oder β-Position zum Stickstoffatom befindet, mit mindestens einer elektronenziehenden Gruppe substituiertes C₆bis C₁₄-Aryl sowie Nitril-, Sulfinyl (-SO₂R⁷), Sulfonyl- (-SO₃R⁷) und Nitrogruppen geeignet. Des weiteren kann R⁵ für -C(O)R⁸ stehen, wobei R⁸ für lineares oder verzweigtes C₁- bis C₂₀-Alkyl, C₆- bis C₁₄-Aryl oder Aralkyl mit 1 bis 10 C-Atomen im Alkylteil und 6 bis 14 C-Atomen im Arylteil, lineares oder verzweigtes C₁bis C₂₀-Alkyl, das mit mindestens einer elektronenziehenden Gruppe substituiert ist, wobei sich die elektronenziehende Gruppe vorzugsweise in α-, β-, γ- und/oder δ-Position zur C(O)-Gruppe befindet, sowie C₆- bis C₁₄-Aryl, das mit mindestens einer elektronenziehenden Gruppe substituiert ist, steht. Bevorzugt als Reste R⁸ sind u.a. Methyl, Ethyl, i-Propyl, Phenyl, Trifluormethylphenyl, Trifluormethyl oder Pentafluorethyl.

Als elektronenziehende Substituenten an einem Alkyl- oder Arylrest R⁵ kommen Halogenatome, wie Fluor, Chlor oder Brom, vorzugsweise Fluor und Chlor, und besonders bevorzugt Fluor, in Frage. Die Alkyl- und Arylreste können sowohl partiell als auch perhalogeniert sein. Weiterhin sind die Nitro-, Nitril-, Ester-, Amid-, Sulfinyl-, Sulfonylamid- und Sulfonylgruppe als Substituenten für die Alkyl- und Arylreste geeignet. Die Arylreste können auch mit Trifluor- oder Trichlormethylgruppen sowie mit Ammoniumresten substituiert sein. Beispielhaft seien als geeignete Alkylreste R⁵ genannt: Trifluormethyl, Trichlormethyl, Difluormethyl, 2,2,2-Trifluorethyl, 2,2,2-Trichlorethyl, 3,3,3-Trifluorpropyl, Pentafluorethyl, Nitromethyl, 2-Nitromethyl, 2-Nitroethyl und Cyanomethyl. Bevorzugt sind Trifluormethyl und 2,2,2-Trifluorethyl. Unter den geeigneten Arylresten R⁵ seien beispielhaft genannt: p-, m-, o-Fluor- oder Chlorphenyl, 2,4-Difluorphenyl, 2,4-Dichlorphenyl, 2,4,6-Trifluorphenyl, Pentafluorphenyl, 2,4,6-Trichlorphenyl, Nitrophenyl, 2,4-Dinitrophenyl, 2-Chlor-5-nitrophenyl, 2-Brom-5-nitrophenyl, Methylsulfinylphenyl und Methylsulfonylphenyl.

Die Erfindung betrifft außerdem eine Katalysatorzusammensetzung, die eine Palladium(II)-Verbindung und eine Diphosphinverbindung der allgemeinen Strukturformel R¹R²P-CH₂-N(R⁵)-CH₂-PR³R⁴ umfasst, wobei R¹ bis R⁵ die bereits angegebenen Bedeutungen und bevorzugten Bedeutungen besitzen. In besonders bevorzugten Ausführungsformen der erfindungsgemäßen Katalysatorzusammensetzung stehen R¹ und R² gemeinsam und R³ und R⁴ gemeinsam unabhängig voneinander für 1,3- und/oder 1,4-Cyclooctylen und R⁵ für 2,4-Difluorphenyl, Pentafluorphenyl oder 2,4,6-Trifluorphenyl.

Hinsichtlich der Palladium(II)-Verbindung wird auf die eingangs gemachten Ausführungen verwiesen. Die Katalysatorzusammensetzung kann eine Anionenquelle enthalten oder mit einer Anionenquelle verwendet werden. Wenn die Palladium(II)-Verbindung das Palladiumsalz einer geeigneten Säure ist, kann diese gleichzeitig die Anionenquelle darstellen.

Derartige Diphosphinverbindungen werden auch als Bis(phosphinomethyl)amine bezeichnet. Die Herstellung einiger Vertreter ist von J. Fawcett, P.A.T. Hoye et al. in J. Chem. Soc. Dalton Trans. 1993, 2563-2567 beschrieben worden.

Einige der Bis(phosphinomethyl)amine sind als solche neu. Die Erfindung betrifft daher ferner Diphosphinverbindungen der allgemeinen Strukturformel

R¹R²P-CH₂-N(R⁵)-CH₂-PR³R⁴,

worin R¹ bis R⁵ die bereits angegebenen Bedeutungen und bevorzugten Bedeutungen besitzen, mit der Maßgabe, dass Verbindungen ausgenommen sind, in denen R¹, R², R³ und R⁴ gleich sind und für Phenyl stehen, und R⁵ für CHMePh, CHMeCO₂Me, CHMeCO₂Et, endo-(1R)-1,7,7-Trimethylbicyclo[2.2.1]heptan-2-yl, CH₂CH₂OH oder CH₂CH=CH₂ steht; R¹, R², R³ und R⁴ gleich sind und für Cyclohexyl stehen und R⁵ für CHMePh oder CHMeCO₂H steht; R¹ und R² gemeinsam sowie R³ und R⁴ gemeinsam für Cyclooctylen stehen und R⁵ für CHMePh steht. (Me bedeutet einen Methyl-, Et einen Ethyl- und Ph einen Phenylrest.)

Die Herstellung der Bis(phosphinomethyl)amine gelingt z. B. nach folgendem allgemeinen Reaktionsschema

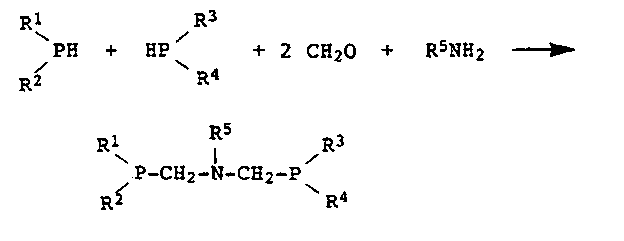

Die Reaktionsbedingungen können zweckmäßigerweise den in J. Fawcett, P.A.T. Hoye, et al. , J. Chem. Soc. Dalton Trans. 1993, 2563-2567 beschriebenen analog gewählt werden. So können die Bis(phosphinomethyl)amine leicht in einer Eintopfreaktion durch Umsetzung des sekundären Phosphans mit Formaldehyd und Ammoniak oder einem primären Amin, z. B. in einem Lösungsmittel, wie Toluol, bei einer Temperatur von vorzugsweise 80 °C bis 150 °C hergestellt werden.

Vorzugsweise liegt das Molverhältnis der Diphosphinverbindung zu der Palladium(II)-Verbindung, bezogen auf Palladium, im Bereich von 0,5 bis 20 zu 1, insbesondere im Bereich von 1 bis 5 zu 1.

Als Anionenquelle kommen Lewis- und Protonensäuren und deren Gemische in Betracht. Vorzugsweise werden als Anionenquellen schwache organische Säuren, z. B. mit einem pKₐ-Wert von 3,5 oder höher, insbesondere sterisch gehinderte organische Säuren, verwendet. Bevorzugte organische Säuren sind z. B. Benzoesäure, 2,4,6-Trimethylbenzoesäure, 2,6-Dichlorbenzoesäure, 9-Anthracencarbonsäure, Pivalinsäure, 1,2,3-Benzoltricarbonsäure und deren Teilester, 2-Ethoxy-1-naphthalincarbonsäure, 2,6-Dimethoxybenzoesäure, Essigsäure, Propionsäure, Buttersäure und/oder Cyanvaleriansäure.

Außerdem können starke Mineralsäuren, wie Schwefelsäure, Perchlorsäuren sowie starke organische Säuren, wie Sulfonsäuren, z. B. Methansulfonsäure, p-Toluolsulfonsäure und Benzolsulfonsäuren sowie Trichlor- und Trifluoressigsäure verwendet werden.

Das Molverhältnis von Anionenquelle zu Palladium(II)-Verbindung ist nicht kritisch. Vorzugsweise liegt das Molverhältnis von Anionenquelle zu Palladium(II)-Verbindung im Bereich von 0,5-100 zu 1, insbesondere im Bereich von 1- 0 zu 1, Äquivalente pro Mol Pd.

Der Einsatz von Cyanvaleriansäure ist besonders bevorzugt, weil die als Nebenreaktion auftretende Veresterung der als Anionenquelle eingesetzten organischen Säure mit der hydroxylgruppenhaltigen Verbindung, z. B. einem Alkohol, nicht zu einer Verunreinigung des erhaltenen Reaktionsproduktes führt.

Bei der hydroxylgruppenhaltigen Verbindung, die beim erfindungsgemäßen Verfahren eingesetzt wird, kann es sich z. B. um Wasser oder einen Alkohol, insbesondere einen Alkohol mit 1 - 6 Kohlenstoffatomen handeln. Als Alkohol kann jeder primäre, sekundäre oder tertiäre Alkohol eingesetzt werden. Beispiele bevorzugter Alkohole sind u.a. Methanol, Ethanol, Propanol, i-Propanol, Butanole, n-Hexanol, n-Octanol, i-Octanol, 2-Ethylhexanol, Cyclohexanol, Benzylalkohol, Phenylethylalkohol, Ethylenglykol, 1,2- und 1,3-Propylenglykol, Neopentylglykol, Trimethylolpropan und Pentaerythritol. Besonders bevorzugt sind Methanol und Ethanol.

Das erfindungsgemäße Verfahren wird zweckmäßigerweise bei einer Temperatur von 40 - 200°C, vorzugsweise 75 - 170°C durchgeführt. Das erfindungsgemäße Verfahren kann zweckmäßigerweise bei einem Druck von 1 - 200 bar vorzugsweise 5 - 70 bar durchgeführt werden. Die Umsetzung kann unter diskontinuierlichen, kontinuierlichen oder semi-kontinuierlichen Bedingungen erfolgen. Die Reaktionszeiten betragen im Allgemeinen 0,5 Stunden bis 10 Stunden.

Das erfindungsgemäß zu verwendende Katalysatorsystem kann homogen oder heterogen sein. Das Katalysatorsystem kann auch in immobilisierter Form verwendet werden. Geeignete Träger sind z. B. Ionenaustauscher, wobei z. B. ionische Gruppen an der Diphosphinverbindung, z. B. bestimmte oben definierte Substituenten Q im Rest R⁵, in Wechselwirkung mit ionischen Gruppen am Ionenaustauscher treten. Die Inmobilisierung kann erfolgen, indem eine Lösung des Katalysatorsystems zum Ionenaustauscher gegeben wird, wobei das Katalysatorsystem haften bleibt.

Das erfindungsgemäße Verfahren wird vorzugsweise in der Flüssigphase durchgeführt. Die Flüssigphase kann vom eingesetzten Pentennitril oder der eingesetzten hydroxygruppenhaltigen Verbindung gebildet werden. Alternativ oder zusätzlich kann sie auch aus einem Lösungsmittel bestehen. Es kann ein beliebiges inertes Lösungsmittel verwendet werden. Beispiele hierfür sind Sulfoxide und Sulfone, z. B. Dimethylsulfoxid, Diisopropylsulfon oder Tetrahydrothiophen-2,2-dioxid, 2-Methylsulfolan, 3-Methylsulfolan, 2-Methyl-4-butylsulfolan; aromatische Kohlenwasserstoffe, wie Benzol, Toluol, Xylol; Ester, wie Methylacetat und Butyrolacton; Ketone, wie Aceton oder Methylisobutylketon; Alkohole, wie Methanol und Ethanol, Ether, wie Tetrahydrofuran, Anisol, 2,5,8-Trioxanonan, Diphenylether und Diisopropylether; Amide, wie Dimethylacetamid und N-Methylpyrrolidon.

Das erfindungsgemäße Verfahren erfolgt vorzugsweise unter weitgehendem Ausschluss von Sauerstoff. Zu diesem Zweck wird das Reaktionsgefäß z. B. mehrfach evakuiert und mit Schutzgas, z. B. Stickstoff, belüftet. Reaktanden, Lösungsmittel und Katalysatorsystem werden unter Luftausschluss zugegeben.

Das erfindungsgemäße Verfahren kann z. B. wie folgt durchgeführt werden: 2-Pentennitril, 3-Pentennitril, 4-Pentennitril oder ein Gemisch hiervon, die hydroxylgruppenhaltige Verbindung, das Katalysatorsystem und gegebenenfalls ein Lösungsmittel werden in einen druckfesten Reaktor eingefüllt. Danach wird Kohlenmonoxid aufgepresst und der Reaktor auf die gewünschte Reaktionstemperatur gebracht. Gegebenenfalls kann durch Nachpressen von Kohlenmonoxid bzw. durch Ablassen von Kohlenmonoxid der Reaktionsdruck eingestellt werden. Nach der Umsetzung kann das Gemisch abgekühlt und entspannt werden. Die Cyanvaleriansäure oder ihre Ester können aus den Gemischen nach üblichen Verfahren, z. B. durch fraktionierte Destillation, isoliert werden.

Das eingesetzte Katalysatorsystem kann unter den Reaktionsbedingungen in situ gebildet werden. Alternativ und bevorzugt wird das Katalysatorsystem jedoch vorab hergestellt. Hierzu werden z. B. die Palladium(II)-Verbindung, der zweizähnige Diphosphinligand und gegebenenfalls eine Anionenquelle jeweils in gegenseitig mischbaren Lösungsmitteln gelöst oder suspendiert und die Lösungen oder Suspensionen vereinigt. Die Palladium(II)-Diphosphin-Komplexverbindung kann dann, gegebenenfalls nach Zugabe eines löslichkeitserniedrigenden Verdünnungsmittels, z. B. durch Filtration isoliert werden.

Die Erfindung wird durch die folgenden Beispiele näher veranschaulicht.

### Beispiel 1

28 mg (0,125 mmol) Palladium(II)-acetat, 155 mg (0,5 mmol) eines Gemisches von 1,3-Bis(1,5-cyclooctylenphosphino)ethan und 1,2-Bis(1,4-cyclooctylenphosphino)ethan und der gemischten Verbindungen mit 1,5- und 1,4-Cyclooctylengruppen (bcope), 445 mg (2,0 mmol) 9-Anthracencarbonsäure und 3 ml (31 mmol) 3-Pentennitril wurden in 10 ml Diphenylether und 5 ml Methanol aufgenommen und in einen 100 ml Autoklaven eingefüllt. Nach Schließen des Autoklaven wurde ein Kohlenmonoxiddruck von 40 bar aufgepresst. Der Autoklav wurde auf 150 °C erhitzt und der Gesamtdruck auf 60 bar eingestellt. Nach der in Tabelle 1 angegebenen Reaktionszeit wurde die Umsetzung durch Abkühlen des Autoklaven beendet. Der Autoklav wurde entspannt und der Flüssigaustrag gaschromatographisch untersucht.

### Beispiel 2

Beispiel 1 wurde wiederholt, wobei jedoch die Umsetzung in einem 300 ml-Autoklaven durchgeführt wurde und folgende Reaktanden in den angegebenen Mengen eingesetzt wurden: 112 mg (0,5 mmol) Palladium(II)-acetat, 845 mg (2,0 mmol) 1,2-Bis(dicyclohexylphosphino)ethan, 2,2 g (10 mmol) 9-Anthracencarbonsäure, 12 ml (124 mmol) 3-Pentennitril, 20 ml Methanol und 40 ml Diphenylether.

### Beispiel 3

Beispiel 1 wurde wiederholt, wobei jedoch folgende Reaktanden in den angegebenen Mengen verwendet wurden: 28 mg (0,125 mmol) Palladium(II)-acetat, 116 mg (0,375 mmol) bcope, 445 mg (2,0 mmol) 9-Anthracencarbonsäure, 10 ml (103 mmol) 3-Pentennitril und 15 ml

Methanol.

### Beispiel 4

Für dieses Beispiel wurde der Palladium(II)-diphosphinkomplex vorab hergestellt.

1,0 g (4,4 mmol) Palladium(II)-acetat wurden in 50 ml Aceton gelöst, 2 Stunden lang bei Raumtemperatur gerührt und über Celit abfiltriert. Zum Filtrat gab man eine Suspension von 2,1 g (4,4 mmol) bcope in 50 ml Aceton und rührte 1 h bei Raumtemperatur. Der entstandene hellgelbe Feststoff (bcope)Pd(OAc)₂) wurde abfiltriert und im Vakuum getrocknet. Ausbeute: 2,2 g (94 %).

Beispiel 1 wurde wiederholt, wobei jedoch folgende Reaktanden in den angegebenen Mengen eingesetzt wurden: 67 mg (0,125 mmol) (bcope)Pd(OAc)₂, 445 mg (2,0 mmol) Anthracencarbonsäure, 10 ml (103 mmol) 3-Pentennitril und 15 ml Methanol. Die Ergebnisse sind jeweils in der nachstehenden Tabelle angegeben.

| Beispiel | Reaktionszeit | Umsatz 3-PN | TON | TOF | Sel. (CVE) | Sel. (5-CVE) |
|---|---|---|---|---|---|---|
| 1 | 6 h | 90 % | 192 | 32 | 90 % | 70 % |
| 2 | 15 h | 40 % | 85 | 14 | 90 % | 71 % |
| 3 | 3 h | 57 % | 470 | 157 | 90 % | 72 % |
| 4 | 3 h | 62 % | 494 | 165 | 90 % | 72 % |
| CVE = Cyanvaleriansäureester | | | | | | |
| TON = Turn-Over-Number | | | | | | |
| TOF = Turn-Over-Frequency | | | | | | |

Der Vergleich von Beispiel 4 mit Beispiel 3 zeigt, dass durch Verwendung des vorab gebildeten, definierten (bcope)Pd(OAc)₂-Komplexes eine Verbesserung der Katalysatoraktivität erreicht wird. Ein weiterer Vorteil in der Verwendung des definierten, vorab gebildeten Komplexes liegt darin, dass ein Überschuss an Diphosphinverbindung nicht erforderlich ist.

### Beispiel 5

Dieses Beispiel veranschaulicht die Herstellung eines Gemisches von 1,3-Bis-(1,4-cyclooctylenphosphinomethyl)phenylamin, 1,3-Bis-(1,5-cyclooctylenphosphinomethyl)phenylamin, 1-(1,4-cyclooctylenphosphinomethyl)-3-(1,5-cyclooctylenphosphinomethyl)phenylamin.

1,2 g (0,035 mol) Paraformaldehyd werden in 100 ml Toluol suspendiert und die Suspension auf 65 °C erwärmt. 1,6 ml (0,0175 mol) Anilin und 5 g (0,035 mol) eines Gemisches von 1,4-Cyclooctenylphosphan und 1,5-Cyclooctenylphosphan werden zugegeben. Nach 5 h erhält man eine klare Lösung, die nach Abkühlen eingeengt wird. Der Rückstand wird in 50 ml Dichlonnethan aufgenommen und das Chelatphosphan durch Zugabe von Ethanol ausgefällt. Nach Filtration wird der weiße Rückstand im Vakuum getrocknet. Ausbeute: 4,8 g (68 %).

### Beispiel 6

Dieses Beispiel veranschaulicht die Herstellung von 1,3-Bis-(1,4-cyclooctylenphosphinomethyl)(2,4-difluorphenyl)amin, 1,3-Bis-(1,5-cyclooctylenphosphinomethyl)(2,4-difluorphenyl)amin, 1-(1,4-cyclooctylenphosphinomethyl)-3-(1,5-cyclooctylenphosphinomethyl)(2,4-difluorphenyl)amin (aza-bcope).

1,2 g (0,035 mol) Paraformaldehyd werden in 100 ml Toluol suspendiert und die Suspension auf 65 °C erwärmt. 1,8 ml (0,0175 mol) 2,4-Difluoranilin und 5 g (0,035 mol) eines Gemisches von 1,4-Cyclooctenylphosphan und 1,5-Cyclooctenylphosphan werden zugegeben und die Lösung über Nacht bei 65 °C gerührt. Nach Abkühlen wird das Lösungsmittel im Vakuum abgezogen, der Rückstand in 20 ml Dichlormethan aufgenommen und das Produkt durch Zugabe von Ether ausgefällt. Der weiße Feststoff wird abfiltriert und im Vakuum getrocknet. Ausbeute: 5,4 g (70 %).

### Beispiel 7

Dieses Beispiel veranschaulicht die Herstellung eines Gemisches der Komplexe 1,3-Bis-(1,4-cyclooctylenphosphinomethyl)(2,4-difluorphenyl)amin-Pd(OAc)₂, 1,3-Bis-(1,5-cyclooctylenphosphinomethyl)(2,4-difluorphenyl)amin-Pd(OAc)₂ und 1-(1,4-cyclooctylenphosphinomethyl)-3-(1,5-cyclooctylenphosphinomethyl)(2,4-difluorphenyl)amin-Pd(OAc)₂.

0,45 g (2,0 mmol) Palladium(II)-acetat wurden in 50 ml Aceton gelöst, 2 h bei Raumtemperatur gerührt und über Celit abfiltriert. Zum Filtrat gab man eine Suspension von 0,88g (2,2 mmol) aza-bcope in 50 ml Aceton und rührte 1 h lang bei Raumtemperatur. Der entstandene hellgelbe Feststoff wurde abfiltriert und im Vakuum getrocknet. Ausbeute: 1,1 g (88 %).

### Beispiel 8

Dieses Beispiel veranschaulicht die Carbonylierung von 3-Pentennitril unter Verwendung des (aza-bcope)Pd(OAc)₂-Komplexes. Beispiel 1 wurde wiederholt, wobei jedoch die folgenden Reaktanden in den angegebenen Mengen eingesetzt wurden: 79 mg (0,125 mmol) (aza-bcope)Pd(OAc)₂ (siehe Beispiel 7), 445 mg (2,0 mmol) Anthracencarbonsäure, 10 ml (103 mmol) 3-Pentennitril und 15 ml Methanol.

| Beispiel | Reaktionszeit | Umsatz 3-PN | TON | TOF | Sel. (CVE) | Sel. (5-CVE) |
|---|---|---|---|---|---|---|
| 8 | 3 h | 75 % | 598 | 200 | 90 % | 71 % |

Der Vergleich von Beispiel 8 mit Beispiel 4 zeigt, dass das Katalysatorsystem (aza-bcope)PD(OAc)₂ eine deutlich höhere Katalysatoraktivität aufweist.

## Patentansprüche

1. Verfahren zur Herstellung von Cyanvaleriansäure oder deren Estern durch Umsetzung von 2-, 3- oder 4-Pentennitril oder Gemischen hiervon mit Kohlenmonoxid und einer Hydroxylgruppen-haltigen Verbindung in Gegenwart eines Katalysatorsystems, das umfasst:
(i) eine Palladium(II)-Verbindung
(ii) einen zweizähnigen Diphosphinliganden und
(iii) eine Anionenquelle.

2. Verfahren nach Anspruch 1, **dadurch gekennzeichnet, dass** der Diphosphinligand folgende allgemeine Strukturformel aufweist:
R¹R²P-X-PR³R⁴
worin R¹, R², R³ und R⁴ unabhängig voneinander für C₁- bis C₂₀-Alkyl, C₃- bis C₁₀-Cycloalkyl, Aryl oder Heteroaryl mit bis zu 4 kondensierten aromatischen Ringen, C₇- bis C₂₀-Aralkyl, die gegebenenfalls substituiert sein können, oder R¹ und R² gemeinsam und/oder R³ und R⁴ gemeinsam für C₂- bis C₂₀-Alkylen, Arylen oder Heteroarylen mit bis zu 4 aromatischen Ringen oder C₇- bis C₂₀-Aralkylen stehen, die gegebenenfalls substituiert sein können, und X für einen zweiwertigen verbrückenden Rest steht, wobei die flankierenden Phosphoratome durch 1 bis 10 Atome getrennt sind.

3. Verfahren nach Anspruch 2, **dadurch gekennzeichnet, dass** R¹, R², R³ und R⁴ unabhängig voneinander jeweils für einen unsubstituierten, gegebenenfalls verzweigten, gegebenenfalls cyclischen Alkylrest mit 1 bis 10 Kohlenstoffatomen stehen oder R¹ und R² gemeinsam und/oder R³ und R⁴ gemeinsam einen gegebenenfalls verzweigten, gegebenenfalls cyclischen Alkylenrest mit 1 bis 10 Kohlenstoffatomen ausbilden.

4. Verfahren nach Anspruch 3, **dadurch gekennzeichnet, dass** R¹, R², R³ und R⁴ ausgewählt sind unter Methyl-, Ethyl-, Propyl-, Isopropyl-, n-Butyl-, s-Butyl, t-Butyl- und/oder Cyclohexylresten oder R¹ und R² gemeinsam und/oder R³ und R⁴ gemeinsam einen Pentamethylen-, Hexamethylen- oder Cyclooctylenrest ausbilden.

5. Verfahren nach einem der Ansprüche 2 bis 4, **dadurch gekennzeichnet, dass** X für eine gegebenenfalls Heteroatome enthaltende Alkylenkette steht.

6. Verfahren nach Anspruch 5, **dadurch gekennzeichnet, dass** X für
-CH₂-N(R⁵)-CH₂-
steht, worin R⁵ für Wasserstoff, C₁- bis C₂₀-Alkyl, gegebenenfalls mit C₁- bis C₆-Alkyl substituiertes C₃- bis C₁₀-Cycloalkyl, gegebenenfalls mit C₁- bis C₆-Alkyl substituiertes C₆bis C₂₀-Aryl, gegebenenfalls mit C₁- bis C₆-Alkyl substituiertes C₇- bis C₂₀-Aralkyl, gegebenenfalls mit C₁- bis C₆-Alkyl substituiertes C₃- bis C₂₀-Heteroaryl, C₂- bis C₂₁-Acyl, wobei die vorgenannten Reste mit Substituenten, ausgewählt unter -NO, -NO₂, -CN, -CO₂⁻, -CO₂R⁶, -CONR⁶₂, Halogen, -NR⁶₂, -OR⁶, -NR⁶₃+, -SO₃⁻, -SO₃R⁶, -SO₂R⁶, SO₂NR⁶₂ und SiR⁷₃ substituiert sein können bzw. in den vorgenannten Resten 1, 2, 3 oder 4 Kohlenstoffatome durch N oder O ausgetauscht sein können, sowie für eine Nitro-, Nitril-, Sulfinyl-, Sulfonylgruppe oder SiR⁷₃ stehen kann, wobei R⁶ für Wasserstoff, C₁- bis C₁₀-Alkyl oder C₆- bis C₁₄-Aryl und R⁷ für C₁- bis C₁₀-Alkyl oder C₆- bis C₁₄-Aryl stehen kann.

7. Verfahren nach einem der vorhergehenden Ansprüche, **dadurch gekennzeichnet, dass** die Anionquelle eine organische Säure mit einem pKₐ-Wert von 3,5 oder höher ist.

8. Diphosphinverbindung der allgemeinen Strukturformel
R¹R²P-CH₂-N(R⁵)-CH₂-PR³R⁴,
worin R¹, R², R³ und R⁴ unabhängig voneinander für C₁- bis C₂₀-Alkyl, C₃- bis C₁₀-Cycloaklyl, Aryl oder Heteroaryl mit bis zu 4 kondensierten aromatischen Ringen, C₇- bis C₂₀-Aralkyl, die gegebenenfalls substituiert sein können, oder R¹ und R² gemeinsam und/oder R³ und R⁴ gemeinsam für C₂- bis C₂₀-Alkylen, Arylen oder Heteroarylen mit bis zu 4 aromatischen Ringen oder C₇- bis C₂₀-Aralkylen stehen, die gegebenenfalls substituiert sein können, und R⁵ für Wasserstoff, C₁- bis C₂₀-Alkyl, gegebenenfalls mit C₁- bis C₆-Alkyl substituiertes C₃- bis C₁₀-Cycloalkyl, gegebenenfalls mit C₁- bis C₆-Alkyl substituiertes C₆- bis C₂₀-Aryl, gegebenenfalls mit C₁- bis C₆-Alkyl substituiertes C₇- bis C₂₀-Aralkyl, gegebenenfalls mit C₁- bis C₆-Alkyl substituiertes C₃- bis C₂₀-Heteroaryl, C₂- bis C₂₁-Acyl, wobei die vorgenannten Reste mit Substituenten, ausgewählt unter -WO, -NO₂, -CN, -CO₂⁻, -CO₂R⁶, -CONR⁶₂, Halogen, -NR⁶₂, -OR⁶, -NR⁶₃⁺, -SO₃⁻, -SO₃R⁶, -SO₂R⁶, SO₂NR⁶₂ und SiR⁷₃ substituiert sein können bzw. in den vorgenannten Resten 1, 2, 3 oder 4 Kohlenstoffatome durch N oder O ausgetauscht sein können, sowie für eine Nitro-, Nitril-, Sulfinyl-, Sulfonylgruppe oder SiR⁷₃ stehen kann, wobei R⁶ für Wasserstoff, C₁- bis C₁₀-Alkyl oder C₆- bis C₁₄-Aryl und R⁷ für C₁- bis C₁₀-Alkyl oder C₆- bis C₁₄-Aryl stehen kann, mit der Maßgabe, dass Verbindungen ausgeschlossen sind, in denen R¹, R², R³ und R⁴ gleich sind und für Phenyl stehen, und R⁵ für CHMePh, CHMeCO₂Me, CHMeCO₂Et, endo-(1R)-1,7,7-Trimethylbicyclo[2.2.1]heptan-2-yl, CH₂CH₂OH oder CH₂CH=CH₂ steht; R¹, R², R³ und R⁴ gleich sind und für Cyclohexyl stehen und R⁵ für CHMePh oder CHMeCO₂H steht; R¹ und R² gemeinsam sowie R³ und R⁴ gemeinsam für Cyclooctylen stehen und R⁵ für CHMePh steht.

9. Katalysatorzusammensetzung, umfassend eine Palladium(II)-Verbindung und eine Diphosphinverbindung der allgemeinen Strukturformel
R¹R²P-CH₂-N(R⁵)-CH₂-PR³R⁴,
worin R¹, R², R³ und R⁴ unabhängig voneinander für C₁- bis C₂₀-Alkyl, C₃- bis C₁₀-Cycloaklyl, Aryl oder Heteroaryl mit bis zu 4 kondensierten aromatischen Ringen, C₇- bis C₂₀-Aralkyl, die gegebenenfalls substituiert sein können, oder R¹ und R² gemeinsam und/oder R³ und R⁴ gemeinsam für C₂- bis C₂₀-Alkylen, Arylen oder Heteroarylen mit bis zu 4 aromatischen Ringen oder C₇- bis C₂₀-Aralkylen stehen, die gegebenenfalls substituiert sein können, und R⁵ für Wasserstoff, C₁- bis C₂₀-Alkyl, gegebenenfalls mit C₁- bis C₆-Alkyl substituiertes C₃- bis C₁₀-Cycloalkyl, gegebenenfalls mit C₁- bis C₆-Alkyl substituiertes C₆- bis C₂₀-Aryl, gegebenenfalls mit C₁- bis C₆-Alkyl substituiertes C₇- bis C₂₀-Aralkyl, gegebenenfalls mit C₁- bis C₆-Alkyl substituiertes C₃- bis C₂₀-Heteroaryl, C₂- bis C₂₁-Acyl, wobei die vorgenannten Reste mit Substituenten ausgewählt unter -NO, -NO₂, -CN, -CO₂⁻, -CO₂R⁶, -CONR⁶₂, Halogen, -NR⁶₂, -OR⁶, -NR⁶₃⁺, -SO₃⁻, -SO₃R⁶, -SO₂R⁶, SO₂NR⁶₂ und SiR⁷₃ substituiert sein können bzw. in den vorgenannten Resten 1, 2, 3 oder 4 Kohlenstoffatome durch Heteroatome ausgetauscht sein können, sowie für eine Nitro-, Nitril-, Sulfinyl-, Sulfonylgruppe oder SiR⁷₃ stehen kann, wobei R⁶ für Wasserstoff, C₁- bis C₁₀-Alkyl oder C₆- bis C₁₄-Aryl und R⁷ für C₁- bis C₁₀-Alkyl oder C₆- bis C₁₄-Aryl stehen kann.

## Claims

1. The process for preparing cyanovaleric acid or esters by reacting 2-, 3- or 4-pentenenitrile or mixtures thereof with carbon monoxide and a hydroxyl compound in the presence of a catalyst system comprising:
(i) a palladium(II) compound
(ii) a bidentate diphosphine ligand, and
(iii) an anion source.

2. The process of claim 1, wherein the diphosphine ligand has the following general structural formula:
R¹R²P-X-PR³R⁴
where R¹, R², R³ and R⁴ are independently C₁-C₂₀-alkyl, C₃-C₁₀-cycloalkyl, aryl or heteroaryl having up to 4 fused aromatic rings, C₇-C₂₀-aralkyl, which may each be substituted, or R¹ and R² and/or R³ and R⁴ are together C₂-C₂₀-alkylene, arylene or hetarylene having up to 4 aromatic rings or C₇-C₂₀-aralkylene, which may each be substituted, and X is a divalent bridging radical such that the flanking phosphorus atoms are separated by from 1 to 10 atoms.

3. The process of claim 2, wherein R¹, R², R³ and R⁴ are each independently an unsubstituted, linear or branched, linear or cyclic alkyl radical having from 1 to 10 carbon atoms, or R¹ and R² and/or R³ and R⁴ are together a linear or branched, linear or cyclic alkylene radical having from 1 to 10 carbon atoms.

4. The process of claim 3, wherein R¹, R², R³ and R⁴ are each selected from methyl, ethyl, propyl, isopropyl, n-butyl, s-butyl, t-butyl and/or cyclohexyl radicals, or R¹ and R² and/or R³ and R⁴ are together a pentamethylene, hexamethylene or cyclooctylene radical.

5. The process of any of claims 2 to 4, wherein X is an alkylene chain which may contain heteroatoms.

6. The process of claim 5, wherein X is
-CH₂-N(R⁵)-CH₂-
where R⁵ is hydrogen, C₁-C₂₀-alkyl, unsubstituted or C₁-C₆-alkyl-substituted C₃-C₁₀-cycloalkyl, unsubstituted or C₁-C₆-alkyl-substituted C₆-C₂₀-aryl, unsubstituted or C₁-C₆-alkyl-substituted C₇-C₂₀-aralkyl, unsubstituted or C₁-C₆-alkyl-substituted C₃-C₂₀-hetaryl, C₂-C₂₁-acyl, the aforementioned radicals may be substituted by substituents selected from the group consisting of -NO, -NO₂, -CN, -CO₂⁻, -CO₂R⁶, -CONR⁶₂, halogen, -NR⁶₂, -OR⁶, -NR⁶₃+, -SO₃⁻, -SO₃R⁶, -SO₂R⁶, SO₂NR⁶₂ and SiR⁷₃, and/or 1, 2, 3 or 4 carbon atoms in the aforementioned radicals may be replaced by N or O, or R⁵ is nitro, nitrile, sulfinyl, sulfonyl or SiR⁷₃, R⁶ is hydrogen, C₁-C₁₀-alkyl or C₆-C₁₄-aryl and R⁷ is C₁-C₁₀-alkyl or C₆-C₁₄-aryl.

7. The process of any of the preceding claims, wherein the anion source is an organic acid having a pKₐ value of 3.5 or higher.

8. A diphosphine compound of the general structural formula
R¹R²P-CH₂-N(R⁵)-CH₂-PR³R⁴,
where R¹, R², R³ and R⁴ are independently C₁-C₂₀-alkyl, C₃-C₁₀-cycloalkyl, aryl or hetaryl having up to 4 fused aromatic rings, C₇-C₂₀-aralkyl, which may each be substituted, or R¹ and R² and/or R³ and R⁴ are together C₂-C₂₀-alkylene, arylene or hetarylene having up to 4 aromatic rings or C₇-C₂₀-aralkylene, which may each be substituted, and R⁵ is hydrogen, C₁-C₂₀-alkyl, unsubstituted or C₁-C₆-alkyl-substituted C₃-C₁₀-cycloalkyl, unsubstituted or C₁-C₆-alkyl-substituted C₆-C₂₀-aryl, unsubstituted or C₁-C₆-alkyl-substituted C₇-C₂₀-aralkyl, unsubstituted or C₁-C₆-alkyl-substituted C₃-C₂₀-heteroaryl, C₂-C₂₁-acyl, the aforementioned radicals may be substituted by substituents selected from the group consisting of -NO, -NO₂, -CN, -CO₂⁻, -CO₂R⁶, -CONR⁶₂, halogen, -NR⁶₂, -OR⁶, -NR⁶₃⁺, -SO₃⁻, -SO₃R⁶, -SO₂R⁶, SO₂NR⁶₂ and SiR⁷₃, and/or 1, 2, 3 or 4 carbon atoms in the aforementioned radicals may be replaced by N or O, or R⁵ is nitro, nitrile, sulfinyl, sulfonyl or SiR⁷₃, R⁶ is hydrogen, C₁-C₁₀-alkyl or C₆-C₁₄-aryl and R⁷ is C₁-C₁₀-alkyl or C₆-C₁₄-aryl, with the proviso that compounds where R¹, R², R³ and R⁴ are each phenyl and R⁵ is CHMePh, CHMeCO₂Me, CHMeCO₂Et, endo-(1R)-1,7,7-trimethylbicyclo[2.2.1]heptan-2-yl, CH₂CH₂OH or CH₂CH=CH₂; R¹, R², R³ and R⁴ are each cyclohexyl and R⁵ is CHMePh or CHMeCO₂H; or R¹ and R² on the one hand and R³ and R⁴ on the other are together cyclooctylene and R⁵ is CHMePh shall be excluded.

9. A catalyst composition comprising a palladium(II) compound and a diphosphine compound of the general structural formula
R¹R²P-CH₂-N(R⁵)-CH₂-PR³R⁴,
where R¹, R², R³ and R⁴ are independently C₁-C₂₀-alkyl, C₃-C₁₀-cycloalkyl, aryl or hetaryl having up to 4 fused aromatic rings, C₇-C₂₀-aralkyl, which may each be substituted, or R¹ and R² and/or R³ and R⁴ are together C₂-C₂₀-alkylene, arylene or hetarylene having up to 4 aromatic rings or C₇-C₂₀-aralkylene, which may each be substituted, and R⁵ is hydrogen, C₁-C₂₀-alkyl, unsubstituted or C₁-C₆-alkyl-substituted C₃-C₁₀-cycloalkyl, unsubstituted or C₁-C₆-alkyl-substituted C₆-C₂₀-aryl, unsubstituted or C₁-C₆-alkyl-substituted C₇-C₂₀-aralkyl, unsubstituted or C₁-C₆-alkyl-substituted C₃-C₂₀-heteroaryl, C₂-C₂₁-acyl, the aforementioned radicals may be substituted by substituents selected from the group consisting of -NO, -NO₂, -CN, -CO₂⁻, -CO₂R⁶, -CONR⁶₂, halogen, -NR⁶₂, -OR⁶, -NR⁶₃⁺, -SO₃⁻, -SO₃R⁶, -SO₂R⁶, SO₂NR⁶₂ and SiR⁷₃, and/or 1, 2, 3 or 4 carbon atoms in the aforementioned radicals may be replaced by heteroatoms, or R⁵ is nitro, nitrile, sulfinyl, sulfonyl or SiR⁷₃, R⁶ is hydrogen, C₁-C₁₀-alkyl or C₆-C₁₄-aryl and R⁷ is C₁-C₁₀-alkyl or C₆-C₁₄-aryl.

## Revendications

1. Procédé de préparation d'acide cyanovalérianique ou de ses esters par réaction de 2-, 3- ou 4-pentènenitrile ou de leurs mélanges avec du monoxyde de carbone et un composé contenant des groupes hydroxyle, en présence d'un système catalytique, qui comporte :
(i) un composé de palladium(II),
(ii) un ligand diphosphonique bidenté, et
(iii) une source d'anions.

2. Procédé suivant la revendication 1, **caractérisé en ce que** le ligand diphosphonique présente la formule structurelle générale suivante :
R¹R²P-X-PR³R⁴
dans laquelle R¹, R², R³ et R⁴ représentent, indépendamment l'un de l'autre, un alkyle en C₁-C₂₀, un cycloalkyle en C₃-C₁₀, un aryle ou un hétéroaryle comportant jusqu'à 4 noyaux aromatiques condensés, un aralkyle en C₇-C₂₀, qui peuvent éventuellement être substitués, R¹ et R² conjointement et/ou R³ et R⁴ conjointement pouvant représenter un alkylène en C₂-C₂₀, un arylène ou un hétéroarylène ayant jusqu'à 4 noyaux aromatiques ou un aralkylène en C₇-C₂₀, qui peuvent éventuellement être substitués, et X représente un radical bivalent formant pont, les atomes de phosphore encadrant étant séparés par 1 à 10 atomes.

3. Procédé suivant la revendication 2, **caractérisé en ce que** R¹, R², R³ et R⁴ représentent, indépendamment l'un de l'autre, un radical alkyle non substitué, éventuellement ramifié, éventuellement cyclique, comportant 1 à 10 atomes de carbone, ou **en ce que** R¹ et R² conjointement et/ou R³ et R⁴ conjointement forment un radical alkylène éventuellement ramifié, éventuellement cyclique, comportant 1 à 10 atomes de carbone.

4. Procédé suivant la revendication 3, **caractérisé en ce que** R¹, R², R³ et R⁴ sont choisis parmi les radicaux méthyle, éthyle, propyle, isopropyle, n-butyle, s-butyle, t-butyle et/ou cyclohexyle, ou **en ce que** R¹et R² conjointement et/ou R³ et R⁴ conjointement forment un radical pentaméthylène, hexaméthytène ou cyclooctylène.

5. Procédé suivant l'une des revendications 2 à 4, **caractérisé en ce que** X représente une chaîne alkylène contenant éventuellement des hétéroatomes.

6. Procédé suivant la revendication 5, **caractérisé en ce que** X représente
-CH₂-N(R⁵)-CH₂-
dans laquelle R⁵ représente de l'hydrogène, un alkyle en C₁-C₂₀, un cycloalkyle en C₃-C₁₀ éventuellement substitué par de l'alkyle en C₁-C₆, un aryle en C₆-C₂₀ éventuellement substitué par de l'alkyle en C₁-C₆, un aralkyle en C₇-C₂₀ éventuellement substitué par de l'alkyle en C₁-C₆, un hétéroaryle en C₃-C₂₀ éventuellement substitué par de l'alkyle en C₁-C₆, un acyle en C₂-C₂₁, les radicaux précités pouvant être substitués par des substituants choisis parmi -NO, -NO₂, -CN, -CO₂⁻, -CO₂R⁶, -CONR⁶₂, halogène, -NR⁶₂, -OR⁶, -NR⁶₃⁺, -SO₃⁻, -SO₃R⁶, -SO₂R⁶, SO₂NR⁶₂ et SiR⁷₃ ou respectivement 1, 2, 3 ou 4 atomes de carbone dans les radicaux précités pouvant être échangés par N ou O, ainsi qu'un groupe nitro, nitrile, sulfinyle, sulfonyle ou SiR⁷₃, R⁶ pouvant représenter de l'hydrogène, un alkyle en C₁-C₁₀ ou un aryle en C₆-C₁₄ et R⁷ un alkyle en C₁-C₁₀ ou un aryle en C₆-C₁₄.

7. Procédé suivant l'une des revendications précédentes, **caractérisé en ce que** la source d'anion est un acide organique présentant une valeur de pKₐ de 3,5 ou plus.

8. Composé diphosphonique de la formule structurelle générale :
R¹R²P-CH₂-N(R⁵)-CH₂-PR³R⁴
dans laquelle R¹, R², R³ et R⁴ représentent, indépendamment l'un de l'autre, un alkyle en C₁-C₂₀, un cycloalkyle en C₃-C₁₀, un aryle ou un hétéroaryle comportant jusqu'à 4 noyaux aromatiques condensés, un aralkyle en C₇-C₂₀, qui peuvent éventuellement être substitués, R¹ et R² conjointement et/ou R³ et R⁴ conjointement pouvant représenter un alkylène en C₂-C₂₀, un arylène ou un hétéroarylène comportant jusqu'à 4 noyaux aromatiques ou un aralkylène en C₇-C₂₀, qui peuvent éventuellement être substitués, et R⁵ représente de l'hydrogène, un alkyle en C₁-C₂₀, un cycloalkyle en C₃-C₁₀ éventuellement substitué par de l'alkyle en C₁-C₆, un aryle en C₆-C₂₀ éventuellement substitué par de l'alkyle en C₁-C₆, un aralkyle en C₇-C₂₀ éventuellement substitué par de l'alkyle en C₁-C₆, un hétéroaryle en C₃-C₂₀ éventuellement substitué par de l'alkyle en C₁-C₆, un acyle en C₂-C₂₁, les radicaux précités pouvant être substitués par des substituants choisis parmi -NO, -NO₂, -CN, -CO₂⁻, -CO₂R⁶, -CONR⁶₂, halogène, -NR⁶₂, -OR⁶, -NR⁶₃⁺, -SO₃⁻, -SO₃R⁶, -SO₂R⁶, SO₂NR⁶₂ et SiR⁷₃ ou respectivement 1, 2, 3 ou 4 atomes de carbone pouvant être échangés dans les radicaux précités par N ou O, ainsi qu'un groupe nitro, nitrile, sulfinyle, sulfonyle ou SiR⁷₃, R⁶ pouvant représenter de l'hydrogène, un alkyle en C₁-C₁₀ ou un aryle en C₆-C₁₄ et R⁷ un alkyle en C₁-C₁₀ ou un aryle en C₆-C₁₄, à la condition que soient exclus des composés dans lesquels R¹, R², R³ et R⁴ sont identiques et représentent du phényle, et R⁵ réprésente CHMePh, CHMeCO₂Me, CHMeCO₂Et, endo-(1R)-1,7,7-triméthylbicyclo-[2.2.1]heptan-2-yle, CH₂CH₂OH ou CH₂CH=CH₂, dans lesquels R¹, R², R³ et R⁴ sont identiques et représentent du cyclohexyle et R⁵ représente CHMePh ou CHMeCO₂H, et dans lesquels R¹ et R² conjointement ainsi que R³ et R⁴ conjointement représentent du cyclooctylène et R⁵ représente CHMePh.

9. Composition catalytique, comprenant un composé de palladium(II) et un composé diphosphonique de la formule structurelle générale :
R¹R²P-CH₂-N(R⁵)-CH₂-PR³R⁴
dans laquelle R¹, R², R³ et R⁴ représentent, indépendamment l'un de l'autre, un alkyle en C₁-C₂₀, un cycloalkyle en C₃-C₁₀, un aryle ou un hétéroaryle comportant jusqu'à 4 noyaux aromatiques condensés, un aralkyle en C₇-C₂₀, qui peuvent éventuellement être substitués, R¹ et R² conjointement et/ou R³ et R⁴ conjointement pouvant représenter un alkylène en C₂-C₂₀, un arylène ou un hétéroarylène comportant jusqu'à 4 noyaux aromatiques ou un aralkylène en C₇-C₂₀, qui peuvent éventuellement être substitués, et R⁵ représente un hydrogène, un alkyle en C₁-C₂₀, un cycloalkyle en C₃-C₁₀ éventuellement substitué par de l'alkyle en C₁-C₆, un aryle en C₆-C₂₀ éventuellement substitué par de l'alkyle en C₁-C₆, un aralkyle en C₇-C₂₀ éventuellement substitué par de l'alkyle en C₁-C₆, un hétéroaryle en C₃-C₂₀ éventuellement substitué par de l'alkyle en C₁-C₆, un acyle en C₂-C₂₁, les radicaux précités pouvant être substitués par des substituants choisis parmi -NO, -NO₂, -CN, -CO₂⁻, -CO₂R⁶, -CONR⁶₂, halogène, -NR⁶₂, -OR⁶, -NR⁶₃⁺, -SO₃⁻, -SO₃R⁶, -SO₂R⁶, SO₂NR⁶₂ et SiR⁷₃ et respectivement 1, 2, 3 ou 4 atomes de carbone pouvant être échangés dans les radicaux précités par des hétéroatomes, ainsi qu'un groupe nitro, nitrile, sulfinyle, sulfonyle ou SiR⁷₃, R⁶ pouvant représenter un hydrogène, un alkyle en C₁-C₁₀ ou un aryle en C₆-C₁₄ et R⁷ un alkyle en C₁-C₁₀ ou un aryle en C₆-C₁₄.
